(19) Europäisches Patentamt
European Patent Office
Office européen des brevets

(11) EP 0 668 281 B1

(12) FASCICULE DE BREVET EUROPEEN

(45) Date de publication et mention de la délivrance du brevet:
**29.03.2000 Bulletin 2000/13**

(21) Numéro de dépôt: **95400314.1**

(22) Date de dépôt: **15.02.1995**

(51) Int Cl.[7]: **C07D 491/04**, C07D 491/14, A61K 31/435 // (C07D491/04, 311:00, 221:00), (C07D491/14, 317:00, 311:00, 221:00)

(54) **Nouveaux analogues de l'acronycine, leur procédé de préparation et les compositions pharmaceutiques qui les contiennent**

Analogen von Acronycine, Verfahren zu ihrer Herstellung und sie enthaltende pharmaceutische Zusammensetzungen

Acronycin analogues, process for their preparation and pharmaceutical compositions containing them

(84) Etats contractants désignés:
**AT BE CH DE DK ES FR GB GR IE IT LI LU NL PT SE**

(30) Priorité: **17.02.1994 FR 9401806**

(43) Date de publication de la demande:
**23.08.1995 Bulletin 1995/34**

(73) Titulaire: **ADIR ET COMPAGNIE
92415 Courbevoie Cédex (FR)**

(72) Inventeurs:
- **Koch, Michel
F-78170 La Celle Saint Cloud (FR)**
- **Tillequin, François
Paris 14ème (FR)**
- **Skaltsounis, Alexios-Leandros
Athenes 11253 (GR)**
- **Rolland, Yves
F-92170 Vanves (FR)**
- **Pierre, Alain
F-78580 Les Alluets Le Roi (FR)**
- **Atassi, Ghanem
F-92400 Saint Cloud (FR)**

(56) Documents cités:
- **DIE PHARMAZIE, vol.46, no.10, Octobre 1991, FRANKFURT AM MAIN page 745 J. REISCH ET AL**
- **CHEMICAL ABSTRACTS, vol. 106, no. 21, 25 Mai 1987, Columbus, Ohio, US; abstract no. 172921z, page 421 ;colonne 1 ; & J. NAT. PROD., vol.49, no.6, 1986 pages 1091 - 1095**
- **CHEMICAL ABSTRACTS, vol. 112, no. 21, 21 Mai 1990, Columbus, Ohio, US; abstract no. 195287k, page 411 ;colonne 2 ; & ANN. PHARM. FR., vol.47, no.3, 1990 pages 149 - 156**
- **CHEMICAL ABSTRACTS, vol. 109, no. 15, 10 Octobre 1988, Columbus, Ohio, US; abstract no. 129396g, page 731 ;colonne 1 ; & PLANTA MED., vol.54, no.1, 1988 pages 24 - 27**
- **CHEMICAL ABSTRACTS, vol. 107, no. 21, 23 Novembre 1987, Columbus, Ohio, US; abstract no. 194911u, page 456 ;colonne 2 ; & HETEROCYCLES, vol.26, no.8, 1987 pages 2057 - 2063**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 668 281 B1

## Description

La présente invention concerne de nouveaux analogues de l'acronycine, leur procédé de préparation et les compositions pharmaceutiques qui les contiennent.

L'acronycine est un alcaloïde isolé pour la première fois en 1948 et dont les propriétés antitumorales ont été mises en évidence dans des modèles expérimentaux en 1966 par G.H. Svoboda et al. (J. Pharmaceut. Sci., 55 (8), (1966), 758-768).

Des études cliniques réalisées sur ce produit par J.H. Scarffe et al. (Cancer Treat. Rep., 67 (1), (1983), 93-94) n'ont donné que peu de réponses, probablement à cause de l'insolubilité de l'acronycine, qui en interdit l'administration par voie intraveineuse, et par la mauvaise biodisponibilité de ce produit en administration orale.

Cependant, les travaux de G.H. Svoboda et al. (Lloydia, 29 (3), (1966), 206-224) mettent en évidence le large spectre d'activité de l'acronycine dans les modèles expérimentaux et notamment sur les tumeurs solides.

Plusieurs composés possédant une structure proche de l'acronycine ont été décrits dans la littérature, pour leurs propriétés antitumorales (Pharmazie, 1991, 46, 745 : Ann. Pharm. Fr., 1989, 47, 149 ; Planta Med., 1988, 54, 24 ; Heterocycles, 1987, 26, 2057 ; J. Nat. Prod., 1986, 49, 1091).

La demanderesse a présentement découvert de nouveaux analogues de l'acronycine plus actifs et plus puissants que celle-ci et en particulier plus solubles de manière à rendre possible l'administration par voie intra-veineuse.

Plus particulièrement, la présente invention concerne les composés de formule (I):

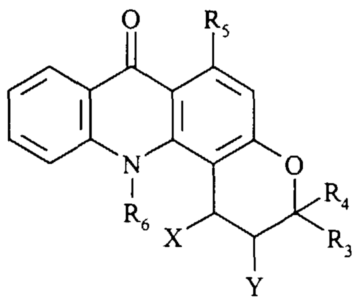

(I)

dans laquelle :

- soit X représente le groupement $-O-R'_1$ et Y représente le groupement

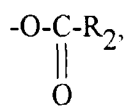

- soit X représente le groupement

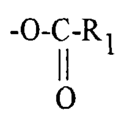

  et Y représente le groupement

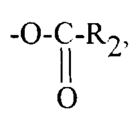

- soit X et Y forment ensemble le groupement

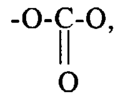

- $R_1$ et $R_2$, identiques ou différents, représentent indépendamment l'un de l'autre un groupement alkyle contenant de 1 à 6 atomes de carbone, en chaîne droite ou ramifiée, et éventuellement substitué par un ou plusieurs groupements choisis parmi hydroxy, halogéno, nitro, amino, alkoxy, alkényloxy, alkynyloxy, acyle, alkénylcarbonyle, et alkynylcarbonyle,

- $R'_1$ est choisi parmi l'hydrogène et $R_1$,

- $R_3$, $R_4$ et $R_6$, identiques ou différents, sont choisis indépendamment l'un de l'autre parmi l'hydrogène et un groupement alkyle, alkényle, alkynyle,

- $R_5$ est choisi parmi l'hydrogène, le groupement hydroxy, et un groupement alkoxy, alkényloxy et alkynyloxy,

étant entendu les termes "alkyle", "alkényle", "alkynyle", "alkoxy", "alkényloxy", "alkynyloxy", "acyle", "alkénylcarbonyle", "alkynylcarbonyle" désignent des groupements contenant de 1 à 6 atomes de carbone en chaîne droite ou ramifiée, et éventuellement substitué par un ou plusieurs groupements choisis parmi hydroxy, halogéno, nitro, amino, alkoxy, alkényloxy, alkynyloxy, acyle, alkénylcarbonyle, alkynylcarbonyle,
leurs éventuels énantiomères, diastéréoisomères, N-oxydes ainsi que, le cas échéant, leurs sels d'addition à un acide ou à une base, pharmaceutiquement acceptables.

La présente invention s'étend également au procédé de préparation des composés de formule (I), caractérisé en ce que l'on fait réagir l'acide anthranilique, en présence d'un chlorure métallique, tel que le chlorure de zinc, en solvant alcoolique anhydre, par exemple le butanol. à reflux, sur le composé de formule (II) :

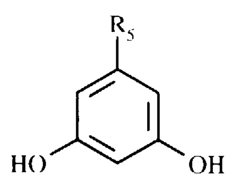

(II)

dans laquelle $R_5$ est tel que défini précédemment,
pour former le composé de formule (III) :

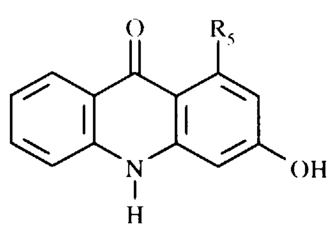

(III)

dans laquelle $R_5$ est tel que défini précédemment,
qui est ensuite traité par un alcyne de formule (IV) :

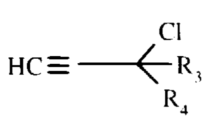

(IV)

dans laquelle $R_3$ et $R_4$ sont tels que définis précédemment,
en solvant aprotique, tel que le diméthylformamide, à reflux, en présence de carbonate alcalin, tel que le carbonate de potassium, pour conduire au composé de formule ($V_1$):

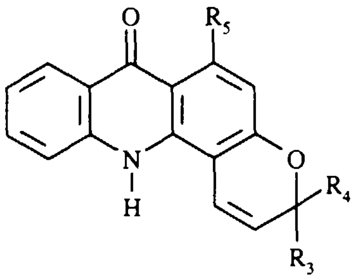

($V_1$)

dans laquelle $R_3$, $R_4$ et $R_5$ sont tels que définis précédemment,
dont l'atome d'azote est éventuellement substitué, par action d'un halogénure d'alkyle ou d'un sulfate de dialkyle en présence d'un agent de déprotonation, tel que l'hydrure de sodium, en solvant polaire aprotique, le diméthylformamide par exemple, de manière à obtenir le composé de formule ($V_2$):

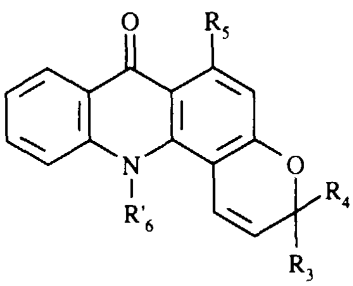

($V_2$)

dans laquelle $R_3$, $R_4$, $R_5$ sont tels que définis précédemment et R'6 est identique à $R_6$ précédemment défini, l'hydrogène excepté,
l'ensemble des composés de formules ($V_1$) et ($V_2$) étant alors soumis à l'action de tétroxyde d'osmium en solvant approprié, tel qu'un mélange tertiobutanol/tétrahydrofurane/eau, afin d'obtenir le *cis*-diol vicinal de formule (VI):

(±) 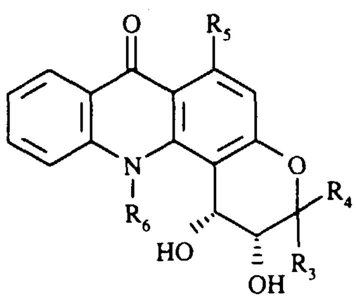 (VI)

dans laquelle $R_3$, $R_4$, $R_5$ et $R_6$ sont tels que définis précédemment,
qui est soumis:

- A/ soit à l'action de N,N'-carbonyldiimidazole de manière à obtenir le composé de formule (I/A):

(±) 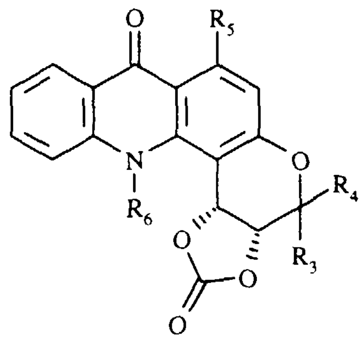 (I/A)

dans laquelle $R_3$, $R_4$, $R_5$ et $R_6$ sont tels que définis précédemment,

- C/ soit à l'action d'un alcool de formule $R_1$-OH dans laquelle $R_1$ est tel que précédemment défini en présence d'un acide, tel que l'acide chlorhydrique, de manière à obtenir le composé de formule (VIII) :

(±) 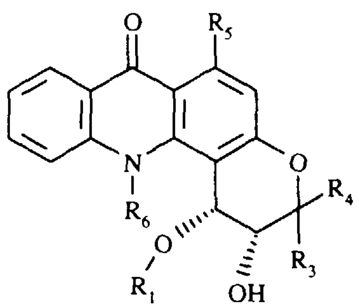 (VIII)

dans laquelle $R_1$, $R_3$, $R_4$, $R_5$ et $R_6$ sont tels que précédemment définis,
dont la fonction alcool libre est estérifiée, en présence d'une base faible, telle que la pyridine, par l'anhydride de formule $(R_2CO)_2O$, dans laquelle $R_2$ est tel que précédemment défini, pour conduire au composé (I/C):

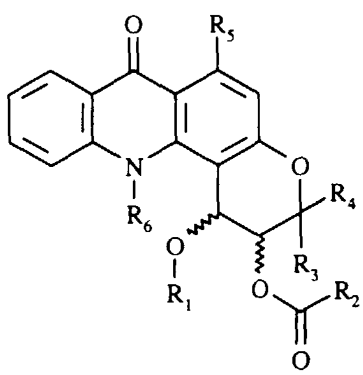 (I/C)

dans laquelle $R_1$, $R_2$, $R_3$, $R_4$, $R_5$ et $R_6$ sont tels que définis précédemment,

- D/ soit directement à l'action de l'anhydride de formule $(R_2CO)_2O$ dans les mêmes conditions opératoires que celles décrites au paragraphe C/, afin d'obtenir le composé de formule (I/D):

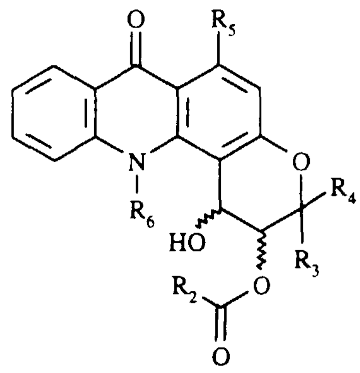

(I/D)

dans laquelle $R_2$, $R_3$, $R_4$, $R_5$ et $R_6$ sont tels que définis précédemment,

qui peut être soumis, dans les mêmes conditions opératoires, à l'action de l'anhydride de formule $(R_1CO)_2O$ pour conduire au composé de formule (I/E) :

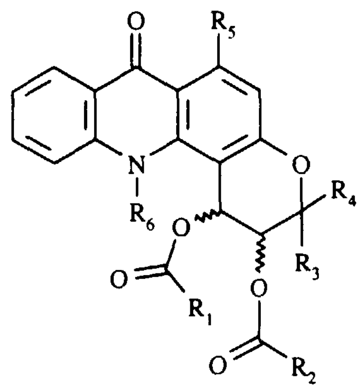

(I/E)

dans laquelle $R_1$, $R_2$, $R_3$, $R_4$, $R_5$ et $R_6$ sont tels que définis précédemment,

l'ensemble des composés de formules (I/A) à (I/E) formant l'ensemble de composés de formule (I) qui sont purifiés et éventuellement séparés en leurs énantiomères et diastéréoisomères par une technique classique de séparation, éventuellement transformés en leurs N-oxydes et, le cas échéant, en leurs sels d'addition à un acide ou à une base pharmaceutiquement acceptables.

Le composé de formule (I) dans lequel X et Y sont identiques et représentent le groupement

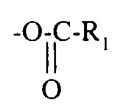

peut être directement obtenu par action de l'anhydre de formule $(R_1CO)_2O$ sur le diol de formule (VI).

Les composés de formule (VI) dans lesquels $R_3$=$R_4$=$CH_3$, peuvent avantageusement être obtenus par action directe du tétroxyde d'osmium sur l'acronycine ($R_5$=OMe et $R_6$=Me), sur la 6-déméthoxyacronycine ($R_5$=H et $R_6$=Me), sur la 6-O-desméthylacronycine ($R_5$=OH et $R_6$=Me) ou sur la N-desméthyl-6-O-desméthylacronycine ($R_5$=OH et $R_6$=H).

Les composés de l'invention présentent, comme l'acronycine des propriétés antitumorales particulièrement intéressantes. Ces nouveaux produits se sont montrés en outre beaucoup plus actifs et plus puissants que le composé de référence. Ils possèdent de plus la propriété d'être solubles et permettent ainsi l'administration par voie intra-veineuse.

La demanderesse propose dans la présente invention l'utilisation des propriétés antitumorales de l'acronycine en réalisant des analogues nouveaux et utilisables en thérapeutique. Les études pharmacologiques présentées dans les exemples suivants montrent le très grand intérêt des nouveaux composés de l'invention pour le traitement de différentes tumeurs aussi bien *in vitro* que *in vivo.*

La présente invention a également pour objet les compositions pharmaceutiques contenant un dérivé de formule (I), ou un de ses N-oxydes ou sels d'addition à un acide ou à une base, pharmaceutiquement acceptables, seul ou en combinaison avec un ou plusieurs excipients inertes et non toxiques. Parmi les compositions pharmaceutiques selon l'invention, on pourra citer plus particulièrement celles qui conviennent à l'administration orale, parentérale, nasale, rectale, perlinguale, oculaire ou pulmonaire, et notamment les préparations injectables, les aérosols, les gouttes oculaires ou nasales, les comprimés simples, dragéifiés ou pelliculés, les gélules, les capsules, les suppositoires, les crèmes, pommades, gels dermiques,...
La posologie utile varie selon l'âge et le poids du patient, la voie d'administration, la nature de l'affection et des traitements éventuels associés et s'échelonne entre 0,2 mg et 2 grammes par 24 heures.
Les exemples suivants illustrent l'invention sans la limiter en aucune façon. Les matières premières sont connues ou préparées à partir de modes opératoires connus.

**Préparation A** : (±)-*cis*-1,2-Dihydroxy-1,2-dihydro-acronycine

3,21 g (10 mmoles) d'acronycine sont ajoutés à un mélange d'une solution de tétroxyde d'osmium à 2,5 % dans 6,4 ml de méthyl-2-propan-2-ol et de 1,68 g (11 mmoles) de dihydrate de N-oxyde de N-méthylmorpholine dans 45 ml d'un mélange tertiobutanol/tétrahydrofurane/eau (10:3:1). Le milieu réactionnel est agité à température ambiante pendant 2 jours. 120 ml d'une solution saturée d'hydrogénosulfate de sodium sont alors ajoutés. Après agitation pendant une heure à température ambiante, le milieu réactionnel est extrait par 5 fois 80 ml de chlorure de méthylène. Le traitement habituel de la phase organique fournit 4 g d'un résidu qui, après purification par flash-chromatographie sur silice (éluant: chlorure de méthylène/méthanol, 98:2 à 95:5) fournit 2,66 g (7,5 mmoles) de produit attendu.
Rendement : 75 %
Caractéristiques spectrales : Infra-rouge (KBr):
ν max ($cm^{-1}$): 3400, 2920, 1650, 1600, 1585, 1390, 1095

**Préparation B** : (±)-*cis*-1,2-Dihydroxy-1,2-dihydro-6-déméthoxy-acronycine

Composé préparé selon le mode opératoire décrit dans la préparation A, à partir de la 6-déméthoxy-acronycine.
Rendement: 65 %
Caractéristiques spectrales : Infra-rouge (KBr):
ν max ($cm^{-1}$): 3450, 3290, 3005, 3000, 2985, 1605, 1555, 770, 650

**Préparation C** : (±)-*cis*-1,2-Dihydroxy-1,2-dihydro-6-O-desméthylacronycine

Composé préparé selon le mode opératoire décrit dans la préparation A, à partir dela 6-O-desméthylacronycine.
Rendement: 70 %
Caractéristiques spectrales : Infra-rouge (KBr):
ν max ($cm^{-1}$): 3515, 3330, 3005, 2985, 1685, 1595, 1155, 840, 772

**Préparation D** : (±)-*cis*-1,2-Dihydroxy-1,2-dihydro-N-desméthyl-6-O-desméthylacronycine

Composé préparé selon le mode opératoire décrit dans la préparation A à partir de la N-desméthyl-6-O-desméthylacronycine.
Rendement: 70 %
Caractéristiques spectrales : Infra-rouge (KBr):
ν max ($cm^{-1}$): 3500, 3310, 3005, 2980, 1690, 1615, 1490, 1345, 775

**EXEMPLE 1: (±)-*cis*-1,2-Diacétoxy-1,2-dihydro-acronycine**

1,775 g (5 mmoles) de composé obtenu à la préparation A sont ajoutés à un mélange préalablement refroidi de 5 ml de pyridine anhydre et de 5 ml d'anhydride acétique. Le mélange est agité à température ambiante pendant 24 heures puis est versé sur 50 ml d'eau glacée.
Le précipité formé est récupéré par filtration, lavé à l'eau puis séché. 2,034 g du composé attendu sont obtenus.
Rendement : 92 %
Caractéristiques spectrales : Infra-rouge (KBr):
ν max ($cm^{-1}$) : 3000, 2950, 2870, 1752, 1639, 1590, 1505, 1245, 1160, 772

**<u>EXEMPLE 2</u>: (±)-*cis*-1,2-Diacétoxy-1,2-dihydro-6-déméthoxy-acronycine**

Composé préparé selon le mode opératoire décrit dans l'exemple 1, à partir du composé obtenu dans la préparation B.
Rendement: 91 %
Caractéristiques spectrales : Infra-rouge (KBr):
ν max ($cm^{-1}$): 3010, 3000, 2970, 1738, 1625, 1595, 1230, 765, 645

**<u>EXEMPLE 3</u>: (±)-*trans*-1,2-Dibenzoyloxy-1,2-dihydro-acronycine**

0,178 g (0,5 mmoles) de composé obtenu à la préparation A sont mis en solution dans 3 ml de pyridine anhydre, puis traités par 1 g (4 mmoles) d'anhydride benzoïque. Le mélange est agité à température ambiante pendant 36 heures, puis évaporé à sec sans chauffer. Le résidu fournit, après chromatographie sur silice (éluant : acétate d'éthyle/toluène, 70:30) 0,0281 g du composé attendu accompagné des composés décrits aux exemples 4 et 5 suivants.
Rendement :10 %
Caractéristiques spectrales : Infra-rouge(KBr):
ν max($cm^{-1}$) : 3100, 3000, 2890, 2795, 1740, 1630, 1270, 1220, 1000, 770, 720

**<u>EXEMPLE 4</u>: (±)-*cis*-1,2-Dibenzoyloxy-1,2-dihydro-acronycine**

Composé obtenu lors de la purification par chromatographie sur colonne de silice du produit brut obtenu à l'exemple 3.
Rendement : 7,5 %
Caractéristiques spectrales : Infra-rouge(KBr):
ν max (cm-1): 3100, 3000, 2890, 2795, 1740, 1630, 1270, 1220, 1000, 770, 720

**<u>EXEMPLE 5</u>: (±)-*cis*-2-Benzoyloxy-1-hydroxy-1,2-dihydro-acronycine**

Composé obtenu lors de la purification par chromatographie sur colonne de silice du produit brut obtenu à l'exemple 3.
Rendement : 40 %
Caractéristiques spectrales : Infra-rouge (KBr):
ν max ($cm^{-1}$): 3350, 3100, 3000, 2795, 1720, 1630, 1600, 1270, 1220, 770, 720

**<u>EXEMPLE 6</u>: (±)-*cis*-1-Acétoxy-2-benzoyloxy-1,2-dihydro-acronycine**

0,092 g (0,2 mmoles) du composé obtenu à l'exemple 5 sont ajoutés à un mélange, préalablement refroidi, de 2,5 ml de pyridine anhydre et de 2,5 ml d'anhydride acétique. Le mélange est agité à température ambiante pendant 48 heures, puis évaporé à sec sous pression réduite sans chauffer. Le résidu fournit, après purification par chromatographie sur colonne de silice (éluant : chlorure de méthylène) 0,1 g de produit attendu.
Rendement : 95 %
Caractéristiques spectrales : Infra-rouge (KBr):
ν max ($cm^{-1}$): 3100, 3005, 2980, 1730, 1630, 1600, 1285, 1225, 770, 720

**<u>EXEMPLE 7</u>: (±)-*cis*-1,2-Carbonyldioxy-1,2-dihydro-acronycine**

A une solution de 0,710 g (2 mmoles) de composé obtenu dans la préparation A dans 50 ml de butan-2-one sont additionnés 1,62 g (10 mmoles) de N,N'-carbonyldiimidazole. L'ensemble est chauffé à reflux pendant 3 heures sous argon, puis repris par une solution aqueuse à 5 % de carbonate de sodium (60 ml), puis extrait par 3 fois 40 ml d'acétate d'éthyle. Le traitement habituel de la phase organique fournit un produit brut qui, parès cristallisation dans le chlorure de méthylène conduit à 0,5 g du composé attendu.
Rendement: 65,5 %
Caractéristiques spectrales : Infra-rouge (KBr):
ν max ($cm^{-1}$): 3015, 3000, 2990, 1805, 1635, 1610, 1590, 770, 710

**EXEMPLE 8: (±)-*trans*-2-Acétoxy-1-méthoxy-dihydro-acronycine**

A une solution de 0,175 g (0,5 mmoles) de composé obtenu à la préparation A dans 10 ml de méthanol à 0°C est ajouté 1 ml d'une solution méthanolique saturée en acide chlorhydrique gazeux.
Le mélange est agité pendant 48 heures à température ambiante, neutralisé par addition d'une résine Amberlite IR 50 OH, puis filtré et évaporé sous pression réduite. Le résidu obtenu constitué majoritairement de (±)-*cis*/*trans*-2-hydroxy-1-méthoxy-1,2-dihydro-acronycine est acétylé par un mélange de 2 ml de pyridine anhydre et de 2 ml d'anhydride acétique. Le milieu réactionnel est agité à température ambiante pendant 48 heures puis évaporé à sec sous pression réduite, sans chauffer. Le résidu fournit, après purification par chromatographie sur colonne de silice (éluant : cyclohexane/acétate d'éthyle, 70:30 à 50:50). 0,042 g du produit attendu ainsi que 0,040 g du composé décrit à l'exemple 9.
Rendement global: 40 %

**EXEMPLE 9: (±)-*cis*-2-Acétoxy-1-méthoxy-1,2-dihydro-acronycine**

Composé obtenu lors de la purification du produit brut décrit dans l'exemple 8.

**ETUDE PHARMACOLOGIQUE**

**Exemple A: Activité in vitro**

Une lignée cellulaire a été utilisée, la leucémie murine L1210. Les cellules sont cultivées dans du milieu de culture RPMI 1640 complet contenant 10 % de serum de veau foetal, 2 mM de glutamine, 50 U/ml de pénicilline, 50 µg/ml de streptomycine et 10 mM d'Hepes, pH : 7,4. Les cellules sont réparties dans des microplaques et exposées aux composés cytotoxiques pendant 4 temps de doublement, soit 48 h. Le nombre de cellules viables est ensuite quantifié par un essai colorimétrique, le Microculture Tetrazolium Assay (J. Carmichael et al., Cancer Res., 47, 936-942, (1987)). Les résultats sont exprimés en $IC_{50}$, concentration en cytotoxique qui inhibe à 50 % la prolifération des cellules traitées. Les résultats obtenus sont rassemblés dans le tableau 1.

Tableau I

| Cytotoxicité pour les cellules L1210 en culture | |
|---|---|
| **Produits** | **Cytotoxicité $IC_{50}$ µM** |
| Exemple 1 | 4.9 |
| Exemple 3 | 3.4 |
| Exemple 5 | 7.1 |
| Exemple 6 | 5.0 |
| Exemple 7 | 0.2 |
| Acronycine | 27.0 |

Tous les produits de l'invention sont beaucoup plus puissants que le composé de référence (acronycine).

**Exemple B: Activité in vivo**

Dans cet exemple, les produits ont été mis en suspension dans du Tween 80, puis dilués dans l'eau. La concentration de Tween est au maximum de 1 % aux plus fortes doses. Les animaux témoins ont reçu le véhicule seul.

B-1 / Activité antitumorale sur la lignée P388

La lignée P388 (leucémie murine) a été fournie par le National Cancer Institute (Frederick, USA). Les cellules tumorales ($10^6$ cellules) ont été inoculées au jour 0 dans la cavité péritonéale de souris B6D2F1 femelles (Iffa Credo, France). Huit à dix souris de 18 à 20 g ont été utulisées par groupe expérimental. Les produits ont été administrés par voie intrapéritonéale au jour 1 ou une fois par jour pendant 4 jours (J1-4), et par voie intraveineuse au jour 1.
L'activité antitumorale est exprimée en % de T/C:

$$T/C\%(souris)= \frac{\text{Temps de survie médian des animaux traités}}{\text{Temps de survie médian des animaux contrôles}} \times 100$$

Le tableau 2 indique l'activité antitumorale obtenue aux doses optimales, pour chaque schéma et voie d'administration.

Tableau 2

| Activité antitumorale sur la lignée P388 | | | | |
|---|---|---|---|---|
| **Produit** | **Schéma** | **Voie** | **Dose optimale (mg/kg)** | **T/C % (survie)** |
| Acronycine | J1 | IP | 200 | 125 |
| | J1-4 | IP | 100 | 136 |
| Exemple 1 | J1 | IP | 25 | 289 |
| | J1-4 | IP | 12.5 | 172 |
| | J1 | IV | 25 | 220 |
| Exemple 5 | J1 | IP | 12.5 | 258 |
| Exemple 7 | J1 | IP | 50 | 269 |

Les produits sont très actifs sur cette tumeur, le composé de l'exemple 1 étant très actif par les voies i.p. et i.v. L'acronycine est faiblement active par voie i.p., et à des doses beaucoup plus fortes. L'acronycine, totalement insoluble, ne peut être testée par voie i.v..

B-2 / Activité antitumorale des composés sur le côlon 38

Le côlon 38 (fourni par le NCI, Frederick, USA) a été greffé sous forme de fragments par voie sous-cutanée (SC) à des souris B6D2F1 femelles. Les produits ont été administrés par voie i.p. à J2 et J9 et l'activité antitumorale a été déterminée à J21 par mesure du volume tumoral (T/C volume, %).

$$T/C\ \%\ (volume) = \frac{\text{Volume tumoral médian des animaux traités}}{\text{Volume tumoral médian des animaux contrôles}} \times 100$$

Les résultats sont rassemblés dans le tableau 3 suivant:

Tableau 3

| Activité antitumorale des composés sur le côlon 38 | | | | |
|---|---|---|---|---|
| **Produit** | **Schéma** | **Voie** | **Dose (mg/kg)** | **T/C médian % (volume tumoral à J21)** |
| Acronycine | J2,9 | i.p. | 100 | 77 |
| | | | 200 | 61 |
| Exemple 1 | J2,9 | i.p. | 6.25 | 12 |
| | | | 12.5 | 8 |
| | | | 25 | 1 |
| 5-Fluoro-uracil | J2,9 | i.p. | 80 | 0 |

Le composé de l'exemple 1 est très actif sur cette tumeur solide très résistante et est autant actif à 25 mg/kg que le 5-fluoro-uracil (molécule de référence dans ce modèle, utilisée en clinique) à 80 mg/kg.

B-3 / Xénogreffe HT-29 chez la souris nude

Les cellules tumorales HT-29 provenant d'un adénocarcinome de côlon humain (American Type Culture Collection, USA) sont inoculées par voie sous-cutanée chez des souris nude femelles (Iffa Credo, France). La tumeur est ensuite amplifiée par passages successifs de fragments tumoraux de 2-3 $mm^3$ implantés bilatéralement par voie sous-cutanée sur les flancs des animaux. Lorsque la tumeur a atteint une taille de 50 $mm^3$ (soit entre 7 et 10 jours après la greffe), les animaux sont randomisés par groupe de 7 à 10 souris et traités selon le protocole indiqué. Les animaux sont pesés et leur tumeur mesurée deux fois par semaine.

Le volume tumoral est calculé selon la formule suivante:

$$Vt = \frac{a.b^2}{2} \qquad a = \text{longueur de la tumeur}$$

$$b = \text{largeur de la tumeur}$$

Les résultats sont exprimés en volume tumoral relatif médian:

$$\frac{\text{Vt médian au temps t } (V_t)}{\text{Vt médian au temps 0 } (V_0)}$$

L'activité antitumorale du produit administré est évaluée par la valeur minimale du T/C médian calculé au temps t:

$$\text{T/C médian \% (volume)} = \frac{(V_t/V_o) \text{ groupe traité}}{(V_t/V_o) \text{ groupe témoin}} \times 100$$

La valeur minimale de ce paramètre est prise en compte au minimum 7 jours après le dernier traitement selon les normes EORTC (European Organization for the Treatment of Cancer).

Les animaux sont traités par voie i.p. à raison d'une administration hebdomadaire du composé de l'exemple 1 pendant 2 semaines. J0 correspond au premier temps de mesure des tumeurs et à la première administration de produit.

Le composé de l'exemple 1 est actif sur cette tumeur très résistante. Le tableau 4 montre le meilleur T/C.

Tableau 4

| **Activité du composé de l'exemple 1 sur la tumeur HT-29 greffée chez la souris nude** | | | | | |
|---|---|---|---|---|---|
| **Produit** | **Dose (mg/kg)** | **Schéma** | **Δ Poids (g) (J10-J0)** | **T/C médian % minimal (jour)** | **Effet antitumoral** |
| Exemple 1 | 6.25 | J0,7 | -1.1 | 46(J32) | + |
| Témoin | - | - | 0 | 100 | |

**<u>Exemple C</u>: Composition pharmaceutique : Comprimés**

Formule de préparation pour 1000 comprimés dosés à 20 mg

| | |
|---|---|
| Composé de l'exemple 1 | 20 g |
| Lactose | 40 g |
| Stéarate de magnésium | 10 g |
| Amidon de blé | 15 g |
| Amidon de maïs | 15 g |
| Silice | 5 g |
| Hydroxypropylcellulose | 5 g |

**Revendications**

1. Composés de formule (I):

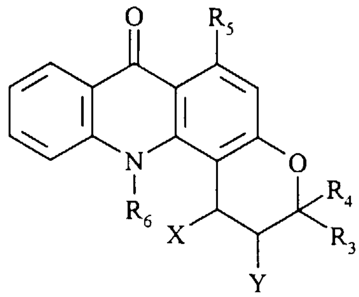

(I)

dans laquelle :

- soit X représente le groupement -O-R'$_1$ et Y représente le groupement

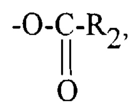

- soit X représente le groupement

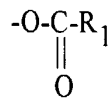

  et Y représente le groupement

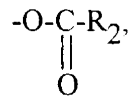

- soit X et Y forment ensemble le groupement

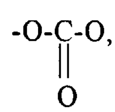

- $R_1$ et $R_2$, identiques ou différents, représentent indépendamment l'un de l'autre un groupement alkyle contenant de 1 à 6 atomes de carbone, en chaîne droite ou ramifiée, et éventuellement substitué par un ou plusieurs groupements choisis parmi hydroxy, halogéno, nitro, amino, alkoxy, alkényloxy, alkynyloxy, acyle, alkénylcarbonyle, et alkynylcarbonyle,
- $R'_1$ est choisi parmi l'hydrogène et $R_1$,
- $R_3$, $R_4$ et $R_6$, identiques ou différents, sont choisis indépendamment l'un de l'autre parmi l'hydrogène et un groupement alkyle, alkényle, alkynyle,
- $R_5$ est choisi parmi l'hydrogène, le groupement hydroxy, et un groupement alkoxy, alkényloxy et alkynyloxy,

étant entendu les termes "alkyle", "alkényle", "alkynyle", "alkoxy", "alkényloxy", "alkynyloxy", "acyle", "alkénylcarbonyle", "alkynylcarbonyle" désignent des groupements contenant de 1 à 6 atomes de carbone en chaîne droite ou ramifiée, et éventuellement substitué par un ou plusieurs groupements choisis parmi hydroxy, halogéno, nitro, amino, alkoxy, alkényloxy, alkynyloxy, acyle, alkénylcarbonyle, alkynylcarbonyle,
leurs éventuels énantiomères, diastéréoisomères, N-oxydes ainsi que, le cas échéant, leurs sels d'addition à un acide ou à une base, pharmaceutiquement acceptables.

**2.** Composés selon la revendication 1 dans laquelle $R_3$ et $R_4$ représentent chacun le radical méthyle,
leurs éventuels énantiomères, diastéréoisomères, N-oxydes ainsi que, le cas échéant, leurs sels d'addition à un acide ou à une base pharmaceutiquement acceptables.

**3.** Composé selon l'une quelconque des revendications 1 à 2 qui est la 1,2-diacétoxy-1,2-dihydro-acronycine,
ses énantiomères, diastéréoisomères, N-oxydes et sels d'addition à un acide, pharmaceutiquement acceptables.

**4.** Composé choisi parmi :

- 1,2-dibenzoyloxy-1,2-dihydro-acronycine
- 2-benzoyloxy-1-hydroxy-1,2-dihydro-acronycine
- 1-acétoxy-2-benzoyloxy-1,2-dihydro-acronycine

leurs énantiomères, diastéréoisomères, N-oxydes et sels d'addition à un acide, pharmaceutiquement acceptables.

**5.** Procédé de préparation des composés de formule (I) selon la revendication 1, caractérisé en ce que l'on fait réagir l'acide anthranilique, en présence d'un chlorure métallique, en solvant alcoolique anhydre, à reflux, sur le composé de formule (II) :

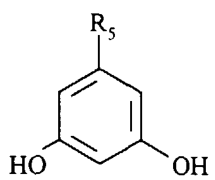

(II)

dans laquelle $R_5$ est tel que défini précédemment,
pour former le composé de formule (III) :

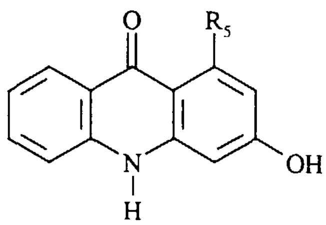

(III)

dans laquelle $R_5$ est tel que défini précédemment,
qui est ensuite traité par un alcyne de formule (IV) :

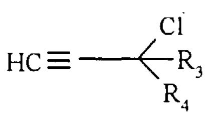

(IV)

dans laquelle $R_3$ et $R_4$ sont tels que définis précédemment,
en solvant aprotique, à reflux, en présence de carbonate alcalin, pour conduire au composé de formule ($V_1$) :

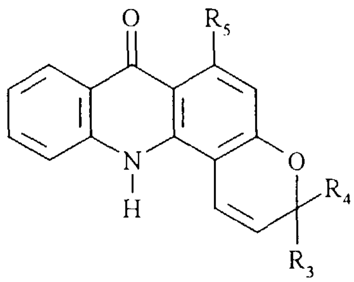

$(V_1)$

dans laquelle $R_3$, $R_4$ et $R_5$ sont tels que définis précédemment, dont l'atome d'azote est éventuellement substitué, par action d'un halogénure d'alkyle ou d'un sulfate de dialkyle, en présence d'un agent de déprotonation, en solvant polaire aprotique, de manière à obtenir le composé de formule $(V_2)$ :

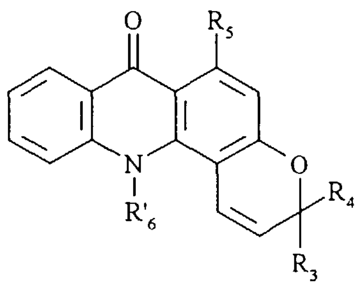

$(V_2)$

dans laquelle $R_3$, $R_4$, $R_5$ sont tels que définis précédemment et $R'_6$ est identique à $R_6$ précédemment défini, l'hydrogène excepté,
l'ensemble des composés de formules $(V_1)$ et $(V_2)$ étant alors soumis à l'action de tétroxyde d'osmium en solvant approprié, afin d'obtenir le *cis*-diol vicinal de formule (VI) :

(±)

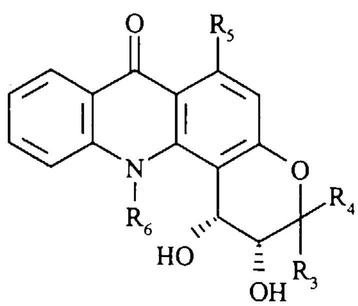

(VI)

dans laquelle $R_3$, $R_4$, $R_5$ et $R_6$ sont tels que définis précédemment,
qui est soumis :

- A/ soit à l'action de N,N'-carbonyldiimidazole de manière à obtenir le composé de formule (I/A) :

(±) 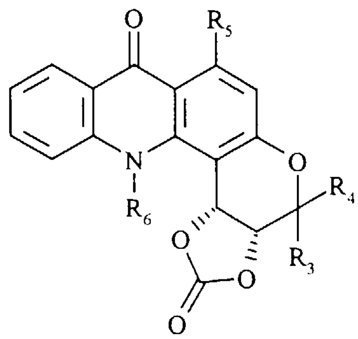 (I/A)

dans laquelle $R_3$, $R_4$, $R_5$ et $R_6$ sont tels que définis précédemment,

- C/ soit à l'action d'un alcool de formule $R_1$-OH dans laquelle $R_1$ est tel que précédemment défini en présence d'un acide, de manière à obtenir le composé de formule (VIII) :

(±) 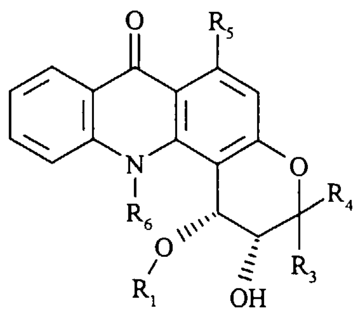 (VIII)

dans laquelle $R_1$, $R_3$, $R_4$, $R_5$ et $R_6$ sont tels que précédemment définis,

dont la fonction alcool libre est estérifiée, en présence d'une base faible, par l'anhydride de formule $(R_2CO)_2O$, dans laquelle $R_2$ est tel que précédemment défini, pour conduire au composé de formule (I/C) :

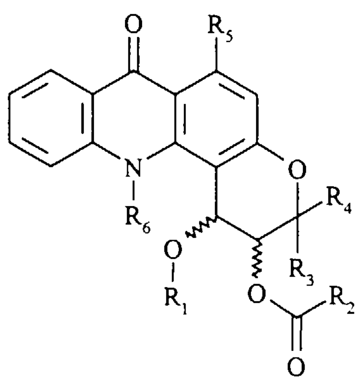 (I/C)

dans laquelle $R_1$, $R_2$, $R_3$, $R_4$, $R_5$ et $R_6$ sont tels que définis précédemment,

- D/ soit directement à l'action de l'anhydride de formule $(R_2CO)_2O$ dans les mêmes conditions opératoires que celles décrites au paragraphe C/, afin d'obtenir le composé de formule (I/D) :

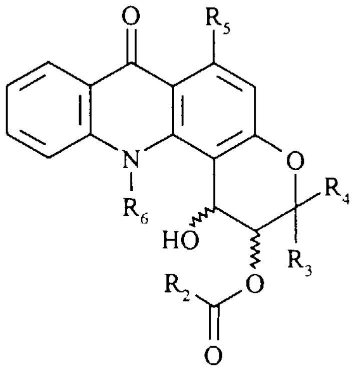

(I/D)

dans laquelle $R_1$, $R_3$, $R_4$, $R_5$ et $R_6$ sont tels que définis précédemment,

qui peut être soumis, dans les mêmes conditions opératoires, à l'action de l'anhydride de formule $(R_2CO)_2O$, pour conduire au composé de formule (I/E) :

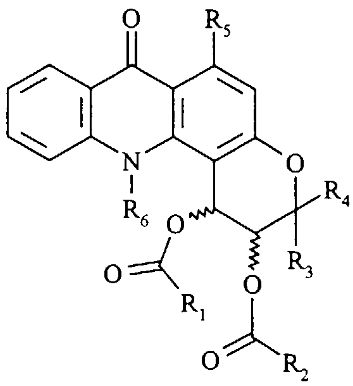

(I/E)

dans laquelle $R_1$, $R_2$, $R_3$, $R_4$, $R_5$ et $R_6$ sont tels que définis précédemment,
l'ensemble des composés de formules (I/A) à (I/E) formant l'ensemble de composés de formule (I) qui sont purifiés et éventuellement séparés en leurs énantiomères et diastéréoisomères par une technique classique de séparation, éventuellement transformés en leurs N-oxydes et, le cas échéant, en leurs sels d'addition à un acide ou à une base pharmaceutiquement acceptables.

**6.** Compositions pharmaceutiques contenant comme principe actif au moins un composé selon l'une quelconque des revendications 1 à 4, seul ou en combinaison avec un ou plusieurs véhicules inertes, non toxiques et pharmaceutiquement acceptables.

**7.** Compositions pharmaceutiques selon la revendication 6 possédant des propriétés antitumorales et utiles dans le traitement de tumeurs cancéreuses.

**Patentansprüche**

**1.** Verbindungen der Formel (I):

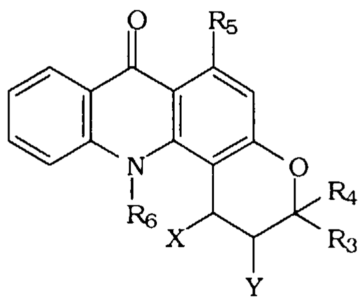

(I)

in der:

- entweder X die Gruppe -O-R'$_1$ <u>und</u> Y die Gruppe

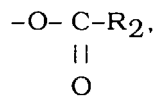

- oder X die Gruppe

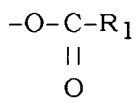

  und Y die Gruppe

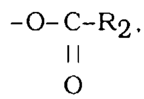

- oder X und Y gemeinsam die Gruppe

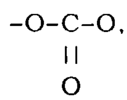

- $R_1$ und $R_2$, die gleichartig oder verschieden sind, unabhängig voneinander eine Alkylgruppe mit 1 bis 6 Kohlenstoffatomen in gerader oder verzweigter Kette, die gegebenenfalls durch eine oder mehrere Gruppen ausgewählt aus Hydroxy, Halogen, Nitro, Amino, Alkoxy, Alkenyloxy, Alkinyloxy, Acyl, Alkenylcarbonyl und Alkinylcarbonyl substituiert sein kann,
- $R'_1$ aus Wasserstoff und $R_1$ ausgewählt ist,
- $R_3$, $R_4$ und $R_6$ gleichartig oder verschieden sind und unabhängig voneinander aus Wasserstoff und Alkyl-, Alkenyl- und Alkinylgruppen ausgewählt sind,
- $R_5$ aus Wasserstoff, Hydroxygruppen und Alkoxy-, Alkenyloxy- und Alkinyloxygruppen ausgewählt ist,

bedeuten, wobei es sich versteht, daß die Begriffe "Alkyl", "Alkenyl", "Alkinyl", "Alkoxy", "Alkenyloxy", "Alkinyloxy", "Acyl", "Alkenylcarbonyl", "Alkinylcarbonyl" für Gruppen stehen, die 1 bis 6 Kohlenstoffatome in gerader oder verzweigter Gruppe enthalten und die gegebenenfalls durch eine oder mehrere Gruppen ausgewählt aus Hydroxy, Halogen, Nitro, Amino, Alkoxy, Alkenyloxy, Alkinyloxy, Acyl, Alkenylcarbonyl und Alkinylcarbonyl substituiert sind, deren eventuelle Enantiomere, Diastereoisomere, N-Oxide sowie gegebenenfalls deren Additionssalze mit einer pharmazeutisch annehmbaren Säure oder Base.

**2.** Verbindungen nach Anspruch 1, worin $R_3$ und $R_4$ jeweils die Methylgruppe bedeuten, deren eventuelle Enantiomere, Diastereoisomere und N-Oxide sowie gegebenenfalls deren Additionssalze mit einer pharmazeutisch annehmbaren Säure oder Base.

**3.** Verbindung nach einem der Ansprüche 1 bis 2, nämlich 1,2-Diacetoxy-1,2-dihydro-acronycin, dessen Enantiomere, Diastereoisomere, N-Oxide und Additionssalze mit einer pharmazeutisch annehmbaren Säure.

**4.** Verbindung ausgewählt aus:

- 1,2-Dibenzoyloxy-1,2-dihydro-acronycin,
- 2-Benzoyloxy-1-hydroxy-1,2-dihydro-acronycin,
- 1-Acetoxy-2-benzoyloxy-1,2-dihydro-acronycin,

deren Enantiomere, Diastereoisomere, N-Oxide und Additionssalze mit einer pharmazeutisch annehmbaren Säure.

**5.** Verfahren zur Herstellung der Verbindungen der Formel (I) nach Anspruch 1, dadurch gekennzeichnet, daß man Anthranilsäure in Gegenwart eines Metallchlorids in einem wasserfreien alkoholischen Lösungsmittel am Rückfluß mit der Verbindung der Formel (II):

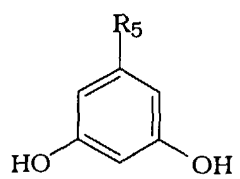

(II)

in der $R_5$ die oben angegebenen Bedeutungen besitzt,
umsetzt zur Bildung der Verbindung der Formel (III):

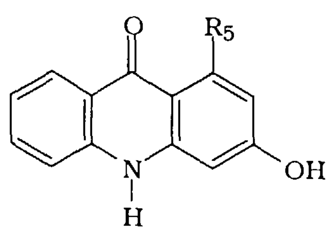

(III)

in der $R_5$ die oben angegebenen Bedeutungen besitzt,
welche man anschließend mit einem Alkin der Formel (IV):

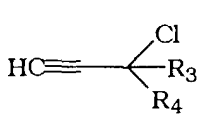

(IV)

in der $R_3$ und $R_4$ die oben angegebenen Bedeutungen besitzen,
in einem aprotischen Lösungsmittel am Rückfluß in Gegenwart eines Alkalimetallcarbonats umsetzt zur Bildung der Verbindung der Formel ($V_1$):

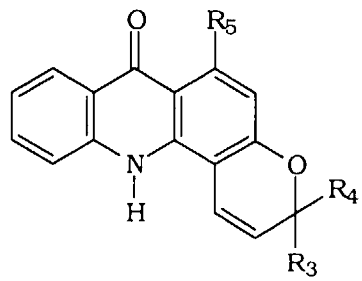

$(V_1)$

in der $R_3$, $R_4$ und $R_5$ die oben angegebenen Bedeutungen besitzen, deren Stickstoffatom gegebenenfalls durch Einwirkung eines Alkylhalogenids oder eines Dialkylsulfats in Gegenwart eines Deprotonierungsmittels in einem polaren aprotischen Lösungsmittel substituiert wird, so daß man die Verbindung der Formel $(V_2)$ erhält:

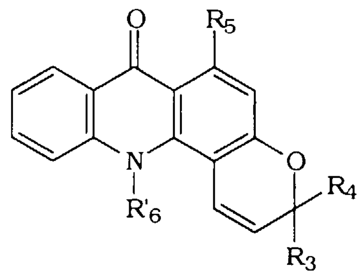

$(V_2)$

in der $R_3$, $R_4$ und $R_5$ die oben angegebenen Bedeutungen besitzen und $R'_6$ identisch ist mit der oben definierten Gruppe $R_6$ mit Ausnahme des Wasserstoffs, wobei die Gesamtheit der Verbindungen der Formeln $(V_1)$ und $(V_2)$ dann der Einwirkung von Osmiumtetroxid in einem geeigneten Lösungsmittel unterworfen wird, so daß man das vicinale cis-Diol der Formel (VI) erhält:

(±)

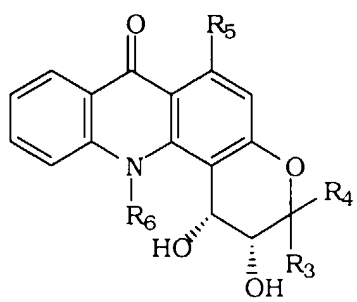

(VI)

in der $R_3$, $R_4$, $R_5$ und $R_6$ die oben angegebenen Bedeutungen besitzen, welches:

- A/ entweder der Einwirkung von N,N'-Carbonyldiimidazol unterworfen wird, so daß man die Verbindung der Formel (I/A) erhält:

(±) 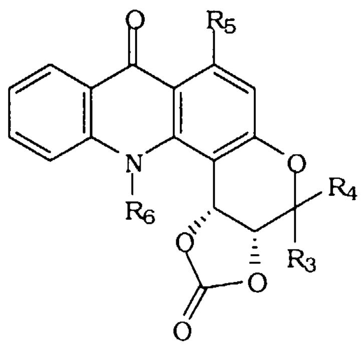 (I/A)

in der $R_3$, $R_4$, $R_5$ und $R_6$ die oben angegebenen Bedeutungen besitzen,

- C/ oder der Einwirkung eines Alkohols der Formel $R_1$-OH, in der $R_1$ die oben angegebenen Bedeutungen besitzt, in Gegenwart einer Säure unterworfen wird, so daß man die Verbindung der Formel (VIII) erhält:

(±) 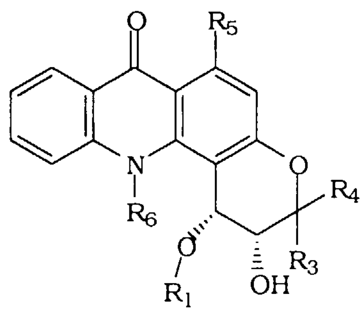 (VIII)

in der $R_1$, $R_3$, $R_4$, $R_5$ und $R_6$ die oben angegebenen Bedeutungen besitzen, deren freie Alkoholfunktion in Gegenwart einer schwachen Base mit dem Anhydrid der Formel $(R_2CO)_2O$, worin $R_2$ die oben angegebenen Bedeutungen besitzt, verestert wird zur Bildung der Verbindung der Formel (I/C):

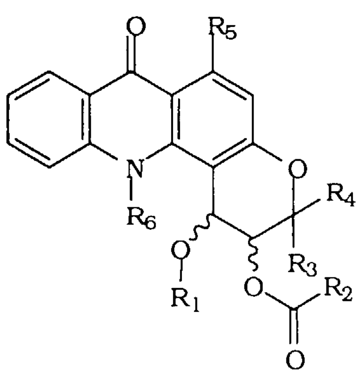 (I/C)

in der $R_1$, $R_2$, $R_3$, $R_4$, $R_5$ und $R_6$ die oben angegebenen Bedeutungen besitzen,

- D/ oder direkt der Einwirkung des Anhydrids der Formel $(R_2CO)_2O$ unter den gleichen Verfahrensbedingungen, wie sie in dem Abschnitt C/ beschrieben worden sind, unterworfen wird zur Bildung der Verbindung der Formel (I/D):

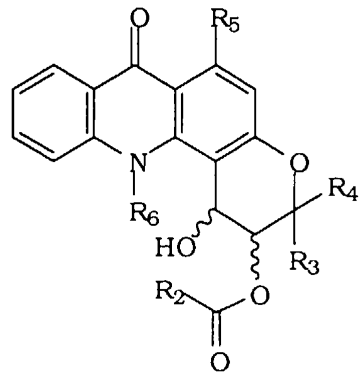

(I/D)

in der $R_2$, $R_3$, $R_4$, $R_5$ und $R_6$ die oben angegebenen Bedeutungen besitzen, welche unter den gleichen Verfahrensbedingungen der Einwirkung des Anhydrids der Formel $(R_1CO)_2O$ unterworfen wird zur Bildung der Verbindung der Formel (I/E):

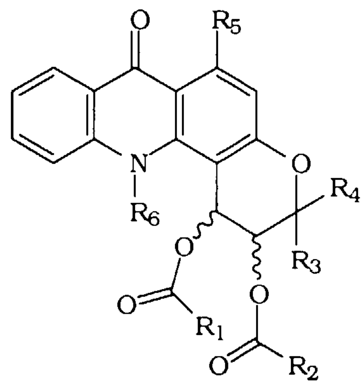

(I/E)

in der $R_1$, $R_2$, $R_3$, $R_4$, $R_5$ und $R_6$ die oben angegebenen Bedeutungen besitzen, wobei die Gesamtheit der Verbindungen der Formeln (I/A) bis (I/E), die die Gesamtheit der Verbindungen der Formel (I) bildet, gereinigt und gegebenenfalls mit Hilfe einer klassischen Trennmethode in ihre Enantiomere und Diastereoisomere aufgetrennt werden können und gegebenenfalls in ihre N-Oxide umgewandelt werden können und gegebenenfalls in ihre Additionssalze mit einer pharmazeutisch annehmbaren Säure oder Base umgewandelt werden können.

**6.** Pharmazeutische Zubereitungen enthaltend als Wirkstoff mindestens eine Verbindung nach einem der Ansprüche 1 bis 4 allein oder in Kombination mit einem oder mehreren inerten, nichttoxischen, pharmazeutisch annehmbaren Trägermaterialien.

**7.** Pharmazeutische Zubereitungen nach Anspruch 6 mit antitumoralen Eigenschaften, die zur Behandlung von Krebstumoren geeignet sind.

## Claims

**1.** Compounds of formula (I) :

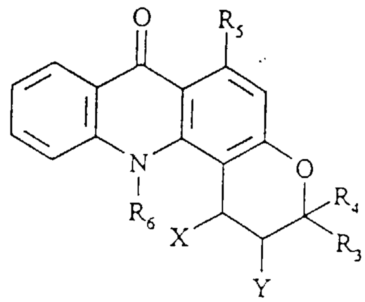

(I)

wherein :

- X represents the group O-R'$_1$ and Y represents the group

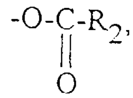

- or X represents the group

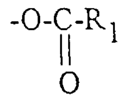

and Y represents the group

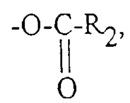

- or X and Y together form the group

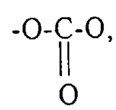

- $R_1$ and $R_2$, which are the same or different, each represents, independently of the other, an alkyl group that contains from 1 to 6 carbon atoms in straight or branched chain and is optionally substituted by one or more groups selected from hydroxy, halogen, nitro, amino, alkoxy, alkenyloxy, alkynyloxy, acyl, alkenylcarbonyl and alkynylcarbonyl,
- $R'_1$ is selected from hydrogen and $R_1$,
- $R_3$, $R_4$ and $R_6$, which are the same or different, are each selected, independently of the others, from hydrogen, an alkyl group, an alkenyl group and an alkynyl group,
- $R_5$ is selected from hydrogen, a hydroxy group, an alkoxy group, an alkenyloxy group and an alkynyloxy group,

the terms "alkyl", "alkenyl", "alkynyl", "alkoxy", "alkenyloxy", "alkynyloxy", "acyl", "alkenylcarbonyl" and "alkynylcarbonyl " being understood to denote groups containing from 1 to 6 carbon atoms in straight or branched chain and optionally substituted by one or more groups selected from hydroxy, halogen, nitro, amino, alkoxy, alkenyloxy, alkynyloxy, acyl, alkenylcarbonyl and alkynylcarbonyl,
their possible enantiomers, diastereoisomers, N-oxides, and also, where appropriate, pharmaceutically acceptable addition salts thereof with an acid or a base.

**2.** Compounds according to claim 1, wherein each of $R_3$ and $R_4$ represents a methyl radical,
their possible enantiomers, diastereoisomers, N-oxides, and also, where appropriate, pharmaceutically acceptable addition salts thereof with an acid or a base.

**3.** Compound according to either claim 1 or 2, which is 1,2-diacetoxy-1,2-dihydro-acronycine,
its enantiomers, diastereoisomers, N-oxides and pharmaceutically acceptable addition salts with an acid.

**4.** Compound selected from :

- 1,2-dibenzoyloxy-1,2-dihydro-acronycine
- 2-benzoyloxy-1-hydroxy-1,2-dihydro-acronycine
- 1-acetoxy-2-benzoyloxy-1,2-dihydro-acronycine,

their enantiomers, diastereoisomers, N-oxides and pharmaceutically acceptable addition salts with an acid.

**5.** Process for the preparation of compounds of formula (I) according to claim 1, characterised in that anthranilic acid is reacted in the presence of a metal chloride, in an anhydrous alcoholic solvent, at reflux, with a compound of formula (II) :

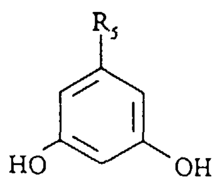

(II)

wherein $R_5$ is as defined above
to form a compound of formula (III) :

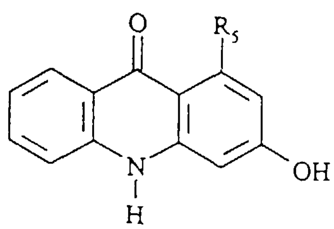

(III)

wherein $R_5$ is as defined above,
which is then treated with an alkyne of formula (IV) :

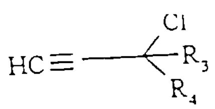

(IV)

wherein $R_3$ and $R_4$ are as defined above,
in an aprotic solvent, at reflux, in the presence of an alkali metal carbonate, to yield a compound of formula ($V_1$) :

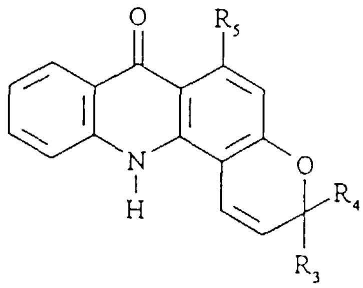

($V_1$)

wherein $R_3$, $R_4$ and $R_5$ are as defined above, the nitrogen atom of which is optionally substituted, by the action of an alkyl halide or a dialkyl sulphate, in the presence of a deprotonation agent, in a polar aprotic solvent, so as to obtain a compound of formula ($V_2$) :

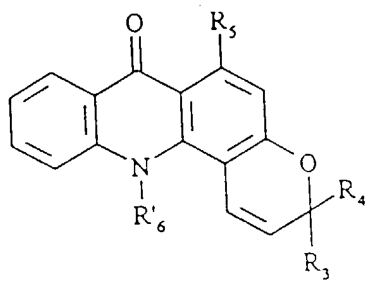

($V_2$),

wherein $R_3$, $R_4$ and $R_5$ are as defined above and $R'_6$ is identical to $R_6$ defined above, with the exception of hydrogen, the totality of the compounds of formulae ($V_1$) and ($V_2$) then being subjected to the action of osmium tetroxide in an appropriate solvent in order to obtain a vicinal cis-diol of formula (VI) :

(±)

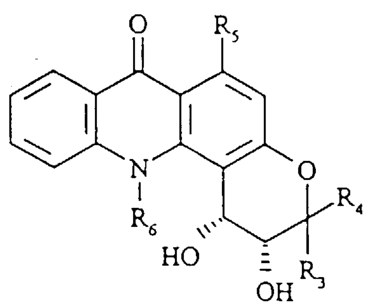

(VI)

wherein $R_3$, $R_4$, $R_5$ and $R_6$ are as defined above, which is subjected :

- A/ either to the action of N,N'-carbonyldiimidazole so as to obtain a compound of formula (I/A) :

(±)

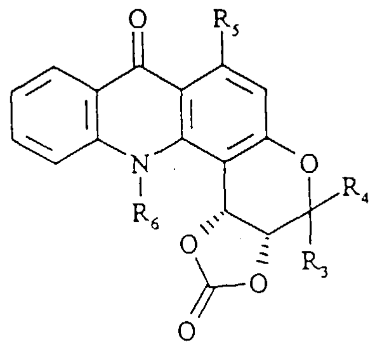

(I/A)

wherein $R_3$, $R_4$, $R_5$ and $R_6$ are as defined above,

- C/ or to the action of an alcohol of formula $R_1$-OH wherein $R_1$ is as defined above, in the presence of an acid, so as to obtain a compound of formula (VIII) :

(±)

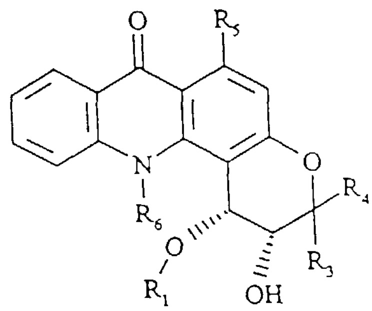

(VIII)

wherein $R_1$, $R_3$, $R_4$, $R_5$ and $R_6$ are as defined above,

the free alcohol function of which is esterified in the presence of a weak base by the anhydride of formula $(R_2CO)_2O$, wherein $R_2$ is as defined above, to yield a compound of formula (I/C) :

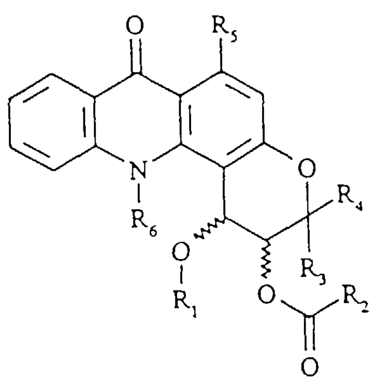

(I/C)

wherein $R_1$, $R_2$, $R_3$, $R_4$, $R_5$ and $R_6$ are as defined above,

- D/ or directly to the action of the anhydride of formula $(R_2CO)_2O$ under the same operating conditions as those described in paragraph C/, in order to obtain a compound of formula (I/D) :

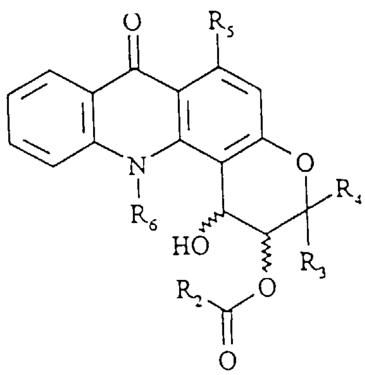

(I/D)

wherein $R_2$, $R_3$, $R_4$, $R_5$ and $R_6$ are as defined above,

which may be subjected, under the same operating conditions, to the action of the anhydride of formula $(R_1CO)_2O$ to yield a compound of formula (I/E) :

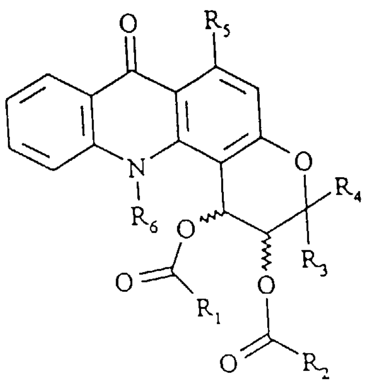

(I/E)

wherein $R_1$, $R_2$, $R_3$, $R_4$, $R_5$ and $R_6$ are as defined above,
the totality of the compounds of formulae (I/A) to (I/E) constituting the totality of the compounds of formula (I), which are purified and, where appropriate, separated into their enantiomers and diastereoisomers by a conventional method of separation,
optionally converted into their N-oxides and, where appropriate, into pharmaceutically acceptable addition salts thereof with an acid or a base.

**6.** Pharmaceutical compositions comprising as active ingredient at least one compound according to any one of claims 1 to 4, alone or in combination with one or more inert, non-toxic and pharmaceutically acceptable carriers.

**7.** Pharmaceutical compositions according to claim 6, possessing anti-tumour properties and useful in the treatment of cancerous tumours.